# EUROPÄISCHE PATENTANMELDUNG

(11) **EP 2 905 041 A1**
(43) Veröffentlichungstag der Anmeldung: **12.08.2015**
(21) Anmeldenummer: 14154732.3
(22) Anmeldetag: 11.02.2014
(51) Int. Cl.: A61M 5/28, A61M 5/32, A61M 5/315

(54) **Spritze**

(71) Anmelder: Sulzer Mixpac AG, 9469 Haag (Rheintal) (CH)
(72) Erfinder: Ettlin, Josef, 9453 Eichberg (CH); Zünd, Marco, 9443 Widnau (CH)
(74) Vertreter: Manitz, Finsterwald & Partner GbR

(57) **Zusammenfassung**

Die Erfindung betrifft eine Spritze (1), umfassend einen Hohlkörper (2), der eine umschließende Hohlkörperfläche (21) aufweist und darin einen Durchgang (22) ausbildet, wobei in eine axiale Längsrichtung des Hohlkörpers (2) eine Achse (A) ausgebildet ist und der Durchgang (22) eine hintere Körperöffnung (23) und eine vordere Körperöffnung (24) aufweist, die entgegengesetzt zueinander in der Längsrichtung angeordnet sind, wobei an der vorderen Körperöffnung (24) ein erster Nadelsitz (25) ausgebildet ist, in welchem eine Nadelkanüle (26) angeordnet und relativ zu welchem die Nadelkanüle (26) entlang der Achse (A) bewegbar ist, einen Kolben (3), der im Hohlkörper (2) angeordnet und in dem Durchgang (22) entlang der Achse (A) bewegbar ist, wobei der Kolben (3) eine vordere Kolbenöffnung (31), ein hinteres Kolbenende (32) und eine umschließende Zwischenwand (33) umfasst, die zwischen der vorderen Kolbenöffnung (31) und dem hinteren Kolbenende (32) angeordnet ist und die eine Aufnahmekammer (34) zur Aufnahme zumindest eines Fluids ausbildet, und ein Zwischenstück (4), das in der Aufnahmekammer (34) angeordnet und entlang der Achse (A) bewegbar ist, wobei zwischen der Zwischenwand (33) und dem Zwischenstück (4) ein erster Dichtbereich (43; 43a) wirksam ist, der die Aufnahmekammer (34) in einer Speicherstellung abdichtet, wobei in dem Zwischenstück (4) ein zweiter Nadelsitz (41) und ein Zuführraum (42) ausgebildet sind, wobei der zweite Nadelsitz (41) und der Zuführraum (42) strömungsverbunden sind, wobei der Kolben (3), der Hohlkörper (2) und das Zwischenstück (4) mittels einer koaxialen Relativbewegung ausgehend von der Speicherstellung in eine Freigabestellung bewegbar sind, und wobei in der Speicherstellung zwischen dem Zuführraum (42) und der Aufnahmekammer (34) eine Abdichtung (47; 43a) vorgesehen ist, die durch die koaxiale Relativbewegung aufhebbar ist, so dass in der Freigabestellung die Aufnahmekammer (34) und der Zuführraum (42) strömungsverbunden sind.

## Beschreibung

Die Erfindung betrifft eine Spritze mit den Merkmalen des Anspruchs 1.

Es sind bereits Spritzen mit einer Nadelkanüle bekannt. Für gewöhnlich besteht die Spritze aus einem Hohlkörper, einem darin beweglichen Kolben und einer konusförmigen Düse. Ferner gibt es Versionen mit einem Schraubgewinde an der Düse, an welche die Nadelkanüle oder ein Schlauch angeschlossen werden kann. Es gibt zweiteilige Spritzen, die nur aus dem Hohlkörper und einem Kolben bestehen, sowie dreiteilige Spritzen, die am unteren Ende des Kolbens noch einen Gummistopfen besitzen. Bei manchen Spritzen, beispielsweise Insulinspritzen, ist die Nadelkanüle eingeklebt. Spritzen können aus Kunststoff, Glas, Metall und Gummi bestehen. Ebenso sind Spritzen mit einer einziehbaren Nadelkanüle bekannt, wobei diese zurückgezogen werden kann, wenn der Kolben am Ende eines Hubs zurückgezogen wird. Nachteil der bekannten Spritze ist die Handhabung. Beispielsweise muss die Nadelkanüle aufwändig an den Hohlkörper montiert werden, wobei die Nadelkanüle durch Bakterien etc. verschmutzt werden kann, wobei außerdem ein Fluid in einer für den Benutzer komplizierten Weise in die Spritze eingezogen werden muss.

Daher ist es eine Aufgabe der vorliegenden Erfindung, eine Spritze zu schaffen, die möglichst einfach zu bedienen ist.

Diese Aufgabe wird durch eine Spritze mit den Merkmalen des Anspruchs 1 gelöst.

Weiterbildungen der Erfindung ergeben sich aus den abhängigen Ansprüchen, der Beschreibung sowie der Zeichnung.

Erfindungsgemäss wird eine Spritze vorgeschlagen, die einen Hohlkörper, einen Kolben und ein Zwischenstück umfasst. Der Hohlkörper weist eine umschließende Hohlkörperfläche auf und bildet darin einen Durchgang aus. In eine axiale Längsrichtung des Hohlkörpers ist eine Achse ausgebildet. Der Durchgang weist eine hintere Körperöffnung und eine vordere Körperöffnung auf, die entgegengesetzt zueinander in der Längsrichtung angeordnet sind, wobei an der vorderen Körperöffnung ein erster Nadelsitz ausgebildet ist, in welchem eine Nadelkanüle angeordnet und relativ zu welchem die Nadelkanüle entlang der Achse bewegbar ist. Der Kolben ist im Hohlkörper angeordnet und in dem Durchgang entlang der Achse bewegbar, wobei der Kolben eine vordere Kolbenöffnung, ein hinteres Kolbenende und eine umschließende Zwischenwand umfasst, die zwischen der vorderen Kolbenöffnung und dem hinteren Kolbenende angeordnet ist und die eine Aufnahmekammer zur Aufnahme zumindest eines Fluids ausbildet. Das Zwischenstück ist in der Aufnahmekammer angeordnet und entlang der Achse bewegbar, wobei zwischen der Zwischenwand und dem Zwischenstück ein erster Dichtbereich wirksam ist, der die Aufnahmekammer in einer Speicherstellung abdichtet. In dem Zwischenstück sind ein zweiter Nadelsitz und ein, insbesondere als Kanal oder kanalartig ausgebildeter, Zuführraum ausgebildet, wobei der zweite Nadelsitz und der Zuführraum strömungsverbunden sind. Der Kolben, der Hohlkörper und das Zwischenstück sind mittels einer koaxialen Relativbewegung ausgehend von einer Speicherstellung in eine Freigabestellung bewegbar, wobei in der Speicherstellung zwischen dem Zuführraum und der Aufnahmekammer eine Abdichtung vorgesehen ist, die durch die koaxiale Relativbewegung aufhebbar ist, so dass in der Freigabestellung die Aufnahmekammer und der Zuführraum strömungsverbunden sind.

Die Spritze kann beispielsweise eine Einwegspritze zur einmaligen Verwendung sein. Ein Material, aus welchem die Spritze hergestellt wird, kann beispielsweise Kunststoff, insbesondere PP oder COC, sein. Mögliche Materialien sind auch PE, PA, PBT und PMMA. Bei dem Material kann es sich aber auch um Glas, um eine metallische Legierung oder um Metall handeln. Vorteilhafterweise kann das Material ein druckstabiles Material sein.

Der Hohlkörper kann ein röhrenförmiger Hohlkörper sein, bevorzugt, aber nicht notwendigerweise, mit einer kreisförmigen, ellipsenförmigen oder mehreckigen Grundfläche. Der Hohlkörper weist eine umschließende Hohlkörperfläche auf, die darin einen Durchgang ausbildet. In eine axiale Längsrichtung des Hohlkörpers ist eine Achse ausgebildet, bevorzugt durch den Schwerpunkt der beiden Grundflächen des Hohlkörpers. Der Hohlkörper kann als ein hauptsächlich hohlzylinderförmiges Rohr ausgeführt sein, wobei der Durchgang eine hintere Körperöffnung und eine vordere Körperöffnung aufweist, die entgegengesetzt zueinander in der Längsrichtung angeordnet sind.

An der vorderen Körperöffnung ist ein erster Nadelsitz ausgebildet, wobei im ersten Nadelsitz eine Nadelkanüle angeordnet und die Nadelkanüle entlang der Achse bewegbar ist. Die vordere Körperöffnung kann beispielsweise düsenformig sein und der erste Nadelsitz kann röhrenförmig in den Hohlkörper hinein mit einer Nadelsitzöffnung ausgebildet sein. Der erste Nadelsitz kann bevorzugt, aber nicht notwendigerweise, eine kreisförmige, ellipsenförmige oder mehreckige Grundfläche aufweisen. Der erste Nadelsitz kann entlang der Achse ausgerichtet sein. In die Nadelkanüle kann aus der Aufnahmekammer Fluid, insbesondere nacheinander ein erstes und ein zweites Fluid, strömen. Die Nadelkanüle kann in der Speicherstellung derart im ersten Nadelsitz angeordnet sein, dass die Nadelkanüle vollständig in den Hohlkörper eingefahren ist. In der Freigabestellung kann die Nadelkanüle aus dem ersten Nadelsitz, also aus dem Hohlkörper, ausgefahren sein, wobei die Nadelkanüle im Zwischenstück gehalten werden kann. In einer Einzugsstellung kann die Nadelkanüle vollständig in den Hohlkörper eingefahren sein und nicht mehr im ersten Nadelsitz angeordnet sein.

Der Kolben ist im Hohlkörper, insbesondere im Durchgang, angeordnet und entlang der Achse bewegbar. Der Kolben umfasst eine vordere Kolbenöffnung, die in Richtung der vorderen Körperöffnung angeordnet ist, und ein hinteres, insbesondere geschlossenes, Kolbenende. Die vordere Kolbenöffnung und das hintere Kolbenende sind entgegengesetzt zueinander in der Längsrichtung angeordnet. Der Kolben kann von der Speicherstellung in die Freigabestellung und umgekehrt bewegt werden. Der Kolben kann außerdem insbesondere in eine Spülstellung und in eine Einzugsstellung bewegt werden, wobei die Spülstellung sich zwischen der Freigabestellung und der Einzugsstellung befindet. Eine Bewegung des Kolbens von der Speicherstellung in die Freigabestellung kann beispielsweise wie bei einer herkömmliches Spritze durch Drücken auf das hintere Kolbenende erfolgen, das beispielsweise als ein Druckknopf ausgebildet sein kann. Damit der Kolben in der Freigabestellung nicht weiter in den Hohlkörper hinein gedrückt werden kann, kann das hintere Kolbenende als eine Anschlageinrichtung, beispielsweise als ein Vorsprung, ausbildet sein. In der Speicherstellung kann der Kolben mittels einer Halteeinrichtung im Hohlkörper gehalten werden, d.h. eine Bewegung des Kolbens kann verhindert werden, und zwar derart, dass eine Bewegung des Kolbens nur ab einer bestimmten Krafteinwirkung auf die hintere Kolbenöffnung möglich ist. Die Halteeinrichtung kann beispielsweise eine Ausbuchtung im Kolben und eine Nut im Hohlkörper, oder umgekehrt, umfassen. Die Ausbuchtung kann beispielsweise in der Nut eingerastet sein. Der Kolben kann auch mittels eines Führungselementes im Durchgang in axialer Richtung geführt sein.

Ebenso umfasst der Kolben eine umschließende Zwischenwand, die zwischen der vorderen Kolbenöffnung und dem hinteren Kolbenende angeordnet ist und die eine Aufnahmekammer zur Aufnahme von Fluid, insbesondere eines ersten und zweiten Fluids, ausbildet. Die Aufnahmekammer kann U-röhrenförmig ausgebildet sein, wobei die Grundfläche der Aufnahmekammer eine kreisförmige, ellipsenförmige oder mehreckige Grundfläche aufweisen kann. Die Aufnahmekammer kann insbesondere ein Volumen von 0,1 bis 100 ml fassen, bevorzugt ein Volumen von 0,5 bis 2 ml.

Im Zwischenstück, das in der Aufnahmekammer angeordnet und entlang der Achse bewegbar ist, ist ein zweiter Nadelsitz ausgebildet, wobei die Nadelkanüle im zweiten Nadelsitz angeordnet und entlang der Achse bewegbar ist. Die Nadelkanüle kann aber auch derart im Zwischenstück, insbesondere in einem inneren Element oder im zweiten Nadelsitz, gehalten sein, dass eine vom Zwischenstück unabhängige koaxiale Bewegung der Nadelkanüle nicht möglich ist. Der zweite Nadelsitz kann entlang der Achse ausgerichtet sein. Die Nadelkanüle kann in der Speicherstellung im ersten und zweiten Nadelsitz angeordnet sein, sodass die Nadelkanüle zumindest annähernd vollständig in den Hohlkörper eingefahren ist. In der Freigabestellung kann die Nadelkanüle aus dem Hohlkörper ausgefahren sein, wobei die Nadelkanüle beispielsweise mittels einer Nadelhalterung im Zwischenstück, insbesondere mittels einer Nadelhalterung im zweiten Nadelsitz, in axialer Richtung festgehalten ist. Die Nadelkanüle kann z.B. mit der Nadelhalterung verrastet oder in die Nadelhalterung eingeklebt oder eingeklemmt sein. Das Zwischenstück kann mittels einer Kraftübertragung vom Kolben auf das Zwischenstück im Durchgang entlang der Achse bewegt werden.

Erfindungsgemäss ist im Zwischenstück ein Zuführraum ausgebildet, der mit dem zweiten Nadelsitz strömungsverbunden ist. Der Zuführraum kann in Form einer oder mehrerer Bohrungen im Zwischenstück vorgesehen sein. Die Bohrung kann z.B. einen größten Durchmesser von 3mm aufweisen und beispielsweise zylinderförmig sein. Eine Querschnittsform der Bohrung senkrecht oder parallel zur Achse kann z.B. rechteckig oder allgemein mehreckig sein. Der Zuführraum kann mit einer Längsachse in einem Winkel von z.B. 50 bis 90 Grad, bevorzugt von zumindest annähernd 90 Grad, zur Achse angeordnet sein. Unter strömungsverbunden ist insbesondere zu verstehen, dass ein Fluid vom Zuführraum in den zweiten Nadelsitz und in die Nadelkanüle strömen bzw. fliessen kann.

Der Kolben, der Hohlkörper und das Zwischenstück sind mittels einer koaxialen Bewegung ausgehend von einer Speicherstellung in eine Freigabestellung relativ zueinander bewegbar. In der Speicherstellung ist die Aufnahmekammer gegenüber dem Zuführraum abgedichtet. Unter der Speicherstellung ist insbesondere zu verstehen, dass der Kolben aus dem Hohlkörper herausgezogen sein kann, sich die vordere Kolbenöffnung also in der Nähe der hinteren Körperöffnung befindet, und durch Abdichten verhindert ist, dass ein Fluid von der Aufnahmekammer in das Zwischenstück und dort in die Nadelkanüle strömt. Unter der Freigabestellung ist zu verstehen, dass der Kolben mittels einer koaxialen Bewegung in den Hohlkörper hineinbewegt, beispielsweise durch Drücken am hinteren Kolbenende, und dadurch die Abdichtung aufgehoben worden ist, so dass das Fluid von der Aufnahmekammer in das Zwischenstück und dort in die Nadelkanüle strömen kann. Dabei kann ausgehend von der Speicherstellung zunächst die Nadelkanüle ausgefahren werden, wobei die Abdichtung noch aktiviert bleibt. Beim Übergang in die Freigabestellung wird die Abdichtung deaktiviert. In einer nachfolgenden Einzugsstellung mit wieder zurückgezogenem Kolben kann ausserdem die Nadelkanüle in den Hohlkörper eingezogen sein.

Vorteile der erfindungsgemässen Spritze sind, dass die Spritze als ein funktionelles Primärpackmittel dient und ein Wirkstoff bereits in der Spritze ist und nicht erst aufgezogen werden muss, wodurch beispielsweise Verunreinigungsprobleme ausgeschlossen werden können. Ausserdem ist die Spritze einfacher zu bedienen, beispielsweise mit einer Hand, weil ein aufwändiges Aufstecken oder Aufschrauben der Nadelkanüle entfällt. Die gegebenenfalls vorgesehene Einzugsstellung hat den Vorteil, dass nach Verwendung der Spritze eine Verletzungs- oder Infektionsgefahr durch die Nadelkanüle ausgeschlossen werden kann.

In einer möglichen Ausgestaltung der Erfindung umfasst das Zwischenstück ein äusseres Element und ein inneres Element. Das innere Element umfasst dabei insbesondere den zweiten Nadelsitz und einen Anschlag für das äussere Element. Das innere Element ist mit dem Anschlag insbesondere derart angeordnet, dass eine axiale Bewegung des inneren Elementes relativ zu dem äusseren Element in Richtung des hinteren Körperendes durch den Anschlag begrenzt ist. Der Zuführraum kann konisch ausgestaltet sein. Zwischen dem inneren Element und dem äusseren Element kann eine Abdichtung ausgebildet sein.

Das Zwischenstück kann einteilig oder mehrteilig sein. Das Zwischenstück kann ein äusseres Element und ein inneres Element umfassen, wobei der zweite Nadelsitz entweder am äusseren Element oder am inneren Element angeordnet sein kann. Das innere Element kann teilweise oder auch vollständig im äusseren Element angeordnet sein. Das innere Element kann insbesondere in einer Bohrung, welche einen kreisförmigen oder mehreckigen Querschnitt aufweisen kann, angeordnet sein. Der Zuführraum kann am inneren Element oder am äusseren Element angeordnet sein. Das innere Element umfasst ausserdem einen Anschlag für das äussere Element, wobei der Anschlag beispielsweise als umlaufende Kante, in Form eines oder mehrerer Stege oder als Stufe ausgebildet sein kann. In der Speicherstellung kann zwischen der Zwischenwand und dem äusseren Element des Zwischenstücks ein erster Dichtbereich und zwischen dem inneren Element und dem äusseren Element ein zweiter Dichtbereich ausgebildet sein. Der erste Dichtbereich kann als eine Dichtung, beispielsweise als eine Nut an der Zwischenwand der Aufnahmekammer und eine Ausbuchtung am Zwischenstück, insbesondere an dem äusseren Element, ausgebildet sein, oder umgekehrt. Der zweite Dichtbereich kann ebenfalls als eine Dichtung, beispielsweise als eine Nut am äusseren Element und eine Ausbuchtung am inneren Element, ausgebildet sein. Der erste Dichtbereich und/oder der zweite Dichtbereichs können alternativ als eine O-Ring-Dichtung ausgebildet sein. Der erste Dichtbereich und/oder der zweite Dichtbereich können eine oder mehrere Dichtungen umfassen. Vorteilhafterweise kann somit der Wirkstoff schon in der Spritze gelagert und ein vereinfachtes Freigabeprinzip des Wirkstoffs erreicht werden.

In einer möglichen Ausgestaltung der Erfindung ist die Aufnahmekammer mittels eines Trennelements in eine erste Kammer und eine zweite Kammer unterteilt. In einer Spülstellung können die zweite Kammer und der Zuführraum strömungsverbunden sein. In der Speicherstellung ist zwischen der Zwischenwand und dem Trennelement ein Dichtbereich derart ausgebildet und angeordnet, dass die zweite Kammer und der Zuführraum keine Strömungsverbindung aufweisen. Das Trennelement kann als eine Folie oder als ein Stopfen, der entlang der Achse beweglich in der Aufnahmekammer angeordnet ist, ausgebildet sein. Das Zwischenstück, bei mehrteiliger Ausführung des Zwischenstücks z.B. ein inneres Element des Zwischenstücks, kann in Richtung des hinteren Körperendes als ein Dorn zum Aufbrechen des Trennelements ausgebildet sein.

Die Aufnahmekammer kann mittels eines Trennelements in genau zwei, nämlich eine erste und eine zweite Kammer, unterteilt sein. Die Aufnahmekammer kann aber auch in mehr als zwei Kammern unterteilt sein, welche mittels mehrerer Trennelemente getrennt sind und insgesamt mehr als zwei Fluide aufnehmen können. Jedes Trennelement kann als eine Folie oder ein Stopfen ausgebildet sein.

Allgemein kann das oder jedes Trennelement aus Kunststoff oder einem metallischen Werkstoff ausgebildet sein. Das Trennelement ist jeweils derart in der Aufnahmekammer angeordnet, dass die betreffenden Kammern fluiddicht getrennt sind. Wenn eine erste Kammer und eine zweite Kammer vorgesehen sind, können diese zwei Fluide aufnehmen, wobei ein erstes Fluid in der ersten Kammer und ein zweites Fluid in der zweiten Kammer angeordnet sein kann. Ausserdem kann in der Speicherstellung oder in der Freigabestellung zwischen der Zwischenwand und dem Trennelement ein Dichtbereich derart ausgebildet und angeordnet sein, dass die zweite Kammer und der Zuführraum sowie die zweite Kammer und die erste Kammer keine Strömungsverbindung aufweisen. Dieser Dichtbereich kann als eine Dichtung, beispielsweise eine Nut in der Zwischenwand und dazu komplementäre Ausbuchtung im Trennelement, oder umgekehrt, ausgebildet sein. Der Dichtbereich kann aber auch ein O-Ring sein. Der Dichtbereich kann eine oder mehrere Dichtungen umfassen. Unter einer Spülstellung ist zu verstehen, dass die zweite Kammer und der Zuführraum strömungsverbunden sind. Das Trennelement oder dessen Position kann zur Herstellung dieser Strömungsverbindung mechanisch verändert worden sein. Beispielsweise kann die Folie mit dem Dorn durchstossen oder der Dichtbereich zwischen dem Trennelement und der Zwischenwand deaktiviert worden sein, so dass die zweite Kammer und der Zuführraum strömungsverbunden sind. In der Speicherstellung und / oder der Freigabestellung hingegen trennt das Trennelement die zweite Kammer und die erste Kammer sowie die zweite Kammer und den Zuführraum derart, dass diese keine Strömungsverbindung aufweisen. Das erste Fluid kann dabei beispielsweise ein zu verabreichendes Medikament und das zweite Fluid eine Spüllösung sein. Von Vorteil hierbei ist, dass das erste Fluid und das zweite Fluid, oder aber auch mehr als zwei Fluide, somit funktionell getrennt werden können. Ausserdem ist die Handhabung und Bedienung der Spritze einfacher, da der Wirkstoff bereits in der Spritze enthalten ist und nicht mehr in die Spritze aufgezogen werden muss.

In einer möglichen Ausgestaltung der Erfindung ist das innere Element des Zwischenstücks als ein weiterer Hohlkörper ausgebildet, wobei der Kolben beweglich im weiteren Hohlkörper angeordnet ist. Der weitere Hohlkörper kann ein röhrenförmiger Hohlkörper sein, bevorzugt, aber nicht notwendigerweise, mit einer kreisförmigen, ellipsenförmigen oder mehreckigen Grundfläche. Die Form des weiteren Hohlkörpers kann der des Hohlkörpers entsprechen, wobei der weitere Hohlkörper im Durchgang des Hohlkörpers angeordnet sein kann. Der weitere Hohlkörper kann einen weiteren Durchgang umfassen und sich entlang der Achse erstrecken. Der weitere Hohlkörper kann als ein hohlzylinderförmiges Rohr ausgeführt sein. Ferner kann der Kolben im weiteren Hohlkörper angeordnet sein. Die koaxiale Bewegung des Kolbens kann entlang der Achse im weiteren Hohlkörper erfolgen, welcher sich wiederum entlang der Achse im Hohlkörper bewegt. Vorteilhafterweise wird so eine bessere Lager- und Führungsstabilität des Zwischenstücks hergestellt.

In einer möglichen Ausgestaltung der Erfindung ist im Durchgang eine Feder angeordnet, gegen deren Rückstellkraft der Kolben in Richtung der vorderen Körperöffnung des Hohlkörpers bewegbar ist. Vorteilhafterweise kann mittels der Feder die Nadelkanüle leichter in den Hohlkörper eingezogen werden, d.h. die Feder kann dafür sorgen, dass die Spritze in die Einzugsstellung gelangt, oder die Feder kann den Übergang in die Einzugsstellung unterstützen.

In einer weiteren möglichen Ausgestaltung der Erfindung sind Mittel zum automatischen seitlichen Verschwenken oder Auslenken des Zwischenstücks in einer Endstellung und/oder Einzugsstellung vorgesehen, wobei insbesondere die Mittel zwischen einem hinteren Ende der Aufnahmekammer und einem hinteren Ende des Zwischenstücks wirksam sind und insbesondere wenigstens eine Schrägfläche umfassen. Durch derartige Mittel ist es möglich, über das Zwischenstück und den zweiten Nadelsitz die Nadelkanüle mit einer Schwenk- oder Auslenkkraft zu beaufschlagen, die danach strebt, die Nadelkanüle aus einer zur Längsachse des Hohlkörpers parallelen Arbeits-Ausrichtung in eine zur Längsachse geneigte Sicherheits-Ausrichtung zu überführen. Die geneigte Stellung kann die Nadelkanüle insbesondere einnehmen, sobald sie außer Eingriff mit dem an der vorderen Körperöffnung ausgebildeten ersten Nadelsitz gelangt, der ansonsten auch gegen die Wirkung der Schwenk- oder Auslenkkraft die Arbeits-Ausrichtung der Nadelkanüle sicherstellt.

Die Erfindung betrifft außerdem eine Spritze wie hierin beschrieben, wobei die Aufnahmekammer bereits wenigstens ein Fluid, insbesondere eine Flüssigkeit enthaltend wenigstens ein zu verabreichendes Medikament, enthält, wobei insbesondere die Aufnahmekammer eine Flüssigkeit enthaltend wenigstens ein zu verabreichendes Medikament in einer ersten Kammer der Aufnahmekammer und eine Spüllösung in einer zweiten Kammer der Aufnahmekammer enthält.

Die Erfindung betrifft des Weiteren eine Verwendung einer Spritze wie hierin beschreiben zum Ausgeben eines bereits in der Aufnahmekammer enthaltenen Fluids ohne Aufziehen des Fluids, insbesondere zum Injizieren einer Flüssigkeit enthaltend wenigstens ein zu verabreichendes Medikament, wobei insbesondere in einer ersten Phase eine Flüssigkeit enthaltend wenigstens ein zu verabreichendes Medikament aus einer ersten Kammer der Aufnahmekammer ausgegeben wird und anschließend in einer zweiten Phase eine Spüllösung aus einer zweiten Kammer der Aufnahmekammer ausgegeben wird.

Weitere Vorteile, Merkmale und Einzelheiten der Erfindung ergeben sich anhand der nachfolgenden Beschreibung von Ausführungsbeispielen sowie anhand der Zeichnung, in welcher gleiche oder funktionsgleiche Elemente mit identischen Bezugszeichen versehen sind. Der Fachmann wird die Merkmale zweckmässigerweise auch einzeln betrachten und zu sinnvollen weiteren Kombinationen zusammenfassen.

Im Folgenden wird die Erfindung anhand von Ausführungsbeispielen unter Bezugnahme auf die Zeichnung näher erläutert. In der schematischen Zeichnung zeigen:
- Fig. 1: eine schematische Darstellung eines ersten Ausführungsbeispiels einer erfindungsgemäßen Spritze in einer Speicherstellung,
- Fig. 2: eine schematische Darstellung des ersten Ausführungsbeispiels in der Freigabestellung,
- Fig. 3: eine schematische Darstellung des ersten Ausführungsbeispiels in einer Endstellung nach Austrag des ersten Fluids und des zweiten Fluids,
- Fig. 4: eine schematische Darstellung des ersten Ausführungsbeispiels in einer Einzugsstellung,
- Fig. 5: eine schematische Darstellung eines zweiten Ausführungsbeispiels in der Speicherstellung,
- Fig. 6: eine schematische Darstellung eines dritten Ausführungsbeispiels in der Speicherstellung,
- Fig. 7: eine schematische Darstellung eines vierten Ausführungsbeispiels in der Speicherstellung,
- Fig. 8: eine schematische Darstellung eines fünften Ausführungsbeispiels in der Speicherstellung.

Fig .1 zeigt eine schematische Darstellung eines ersten Ausführungsbeispiels einer erfindungsgemäßen Spritze 1 in einer Speicherstellung. Die Spritze 1 umfasst einen Hohlkörper 2, einen Kolben 3 und ein Zwischenstück 4. Der Hohlkörper weist eine umschließende Hohlkörperfläche 21 und darin einen Durchgang 22 auf. Der Hohlkörper 21 weist eine kreisförmige Grundfläche auf und bildet einen zylinderförmigen Durchgang 22 aus. In eine axiale Längsrichtung des Hohlkörpers 2 ist eine Achse A ausgebildet. Der Durchgang 22 weist ausserdem eine hintere Körperöffnung 23 und eine vordere Körperöffnung 24 auf, die entgegengesetzt zueinander in der Längsrichtung angeordnet sind.

An der vorderen Körperöffnung 24 ist ein erster röhrenförmiger Nadelsitz 25 ausgebildet, sodass die vordere Körperöffnung 24 bis auf eine Nadelsitzöffnung im ersten Nadelsitz 25 geschlossen ist. Im ersten Nadelsitz 25 ist eine Nadelkanüle 26 angeordnet, die entlang der Achse A relativ zu dem ersten Nadelsitz 25 bewegbar ist.

Der Kolben 3 ist im Hohlkörper 2 angeordnet und im Durchgang 22 entlang der Achse A bewegbar. Ausserdem umfasst der Kolben 3 eine vordere Kolbenöffnung 31, die in Richtung der vorderen Körperöffnung 24 angeordnet ist, und ein hinteres, geschlossenes Kolbenende 32. Die vordere Kolbenöffnung 31 und das hintere Kolbenende 32 sind zueinander entgegengesetzt entlang der Achse A in Längsrichtung angeordnet.

Zwischen der vorderen Kolbenöffnung 31 und dem hinteren Kolbenende 32 ist eine umschließende Zwischenwand 33 angeordnet, die eine Aufnahmekammer 34 zur Aufnahme von Fluid ausbildet. Die Aufnahmekammer 34 ist mittels eines Trennelements 35 in eine erste Kammer 36 und eine zweite Kammer 37 unterteilt. Das Trennelement 35 ist als eine Folie ausgebildet. Die Kammern 36, 37 können jeweils ein Fluid aufnehmen, wobei ein erstes Fluid in der ersten Kammer 36 und ein zweites Fluid in der zweiten Kammer 37 angeordnet ist. In der Speicherstellung trennt die Folie die zweite Kammer 37 von der ersten Kammer 36 und somit von einem im Zwischenstück 4 ausgebildeten Zuführraum 42 derart, dass diese keine Strömungsverbindung aufweisen.

Das erste Fluid kann beispielsweise ein zu verabreichendes Medikament und das zweite Fluid eine Spüllösung sein.

Das Zwischenstück 4 ist in dem Durchgang 22 entlang der Achse A bewegbar angeordnet. Im Zwischenstück 4 ist ein zweiter Nadelsitz 41 ausgebildet, wobei die Nadelkanüle 26 fest im zweiten Nadelsitz 41 gehalten ist. Das Zwischenstück 4 ist zweiteilig und umfasst ein äusseres Element 45 und ein inneres Element 44, wobei der zweite Nadelsitz 41 am inneren Element 44 ausgebildet ist. Das innere Element 44 umfasst ausserdem einen Anschlag 46 für das äussere Element 45, der als umlaufende Schulter ausgebildet ist. Der Anschlag 46 ist in Richtung der vorderen Körperöffnung 24 angeordnet und als ein Stopper für das äussere Element 45 wirksam, der also eine Bewegung des äusseren Elements 45 abstoppen kann. Ausserdem ist das innere Element 44 in Richtung der hinteren Körperöffnung 23 als ein Dorn 48 zum Aufbrechen des Trennelements 35 ausgebildet. Im Weiteren weist das Zwischenstück 4 den Zuführraum 42 auf, der im Zwischenstück 4 ausgebildet ist und der insbesondere konisch ausgestaltet sein kann. Der zweite Nadelsitz 41 und der Zuführraum 42 sind strömungsverbunden, sodass das erste Fluid und das zweite Fluid über den Zuführraum 42 in die Nadelkanüle 26 strömen können, wenn in der Speicherstellung wirksame Abdichtungen deaktiviert sind.

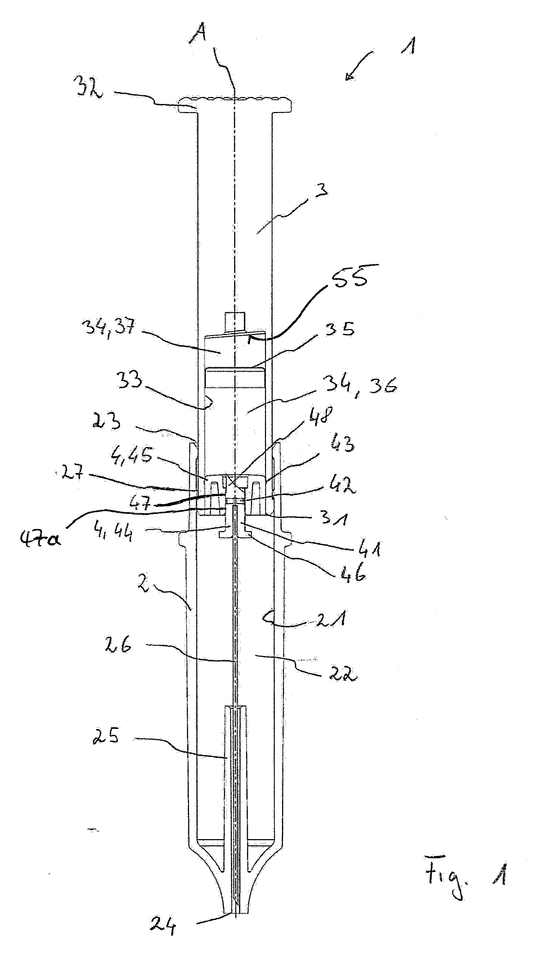

Der Kolben 3, der Hohlkörper 2 und das Zwischenstück 4 sind mittels einer koaxialen Relativbewegung ausgehend von einer Speicherstellung in eine Freigabestellung bewegbar. In der in Fig. 1 vorliegenden Speicherstellung ist zwischen der Zwischenwand 33 und dem äusseren Element 45 des Zwischenstücks 4 ein erster Dichtbereich 43 und zwischen dem inneren und äusseren Element 44, 45 ein weitere Abdichtungen 47, 47a bildender zweiter Dichtbereich ausgebildet, wobei in axialer Richtung betrachtet eine Abdichtung 47 vor dem Zuführraum 42 und eine Abdichtung 47a hinter dem Zuführraum 42 vorgesehen ist. Der erste Dichtbereich 43 umfasst eine Nut an der Zwischenwand 33 der Aufnahmekammer 34 und eine Ausbuchtung am Zwischenstück 4 bzw. dem äusseren Element 45. Der zweite Dichtbereich 47, 47a umfasst jeweils eine weitere Nut am äusseren Element 45 und eine Ausbuchtung am inneren Element 44.

Der erste Dichtbereich 43 verhindert, dass Fluid aus der Aufnahmekammer 34 in den Durchgang 22 des Hohlkörpers 2 strömen kann. Der zweite Dichtbereich verhindert einerseits mittels der Abdichtung 47, dass Fluid in der Speicherstellung in den Zuführraum 42 strömen kann, und andererseits mittels der Abdichtung 47a, dass Fluid zwischen dem inneren Element 44 und dem äusseren Element 45 hindurch in den Durchgang 22 des Hohlkörpers 2 strömen kann. Somit sind die Aufnahmekammer 34 und der Zuführraum 42 in der Speicherstellung nicht strömungsverbunden. Es strömen also weder das erste Fluid noch das zweite Fluid von der Aufnahmekammer 34 in den Zuführraum 42.

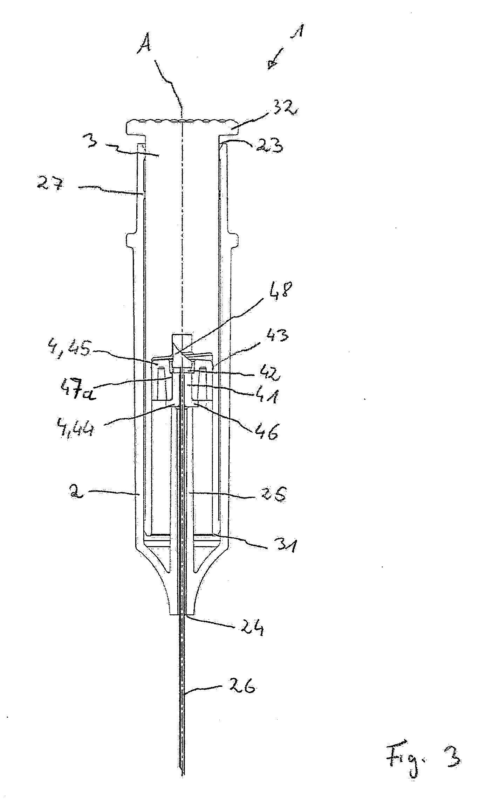

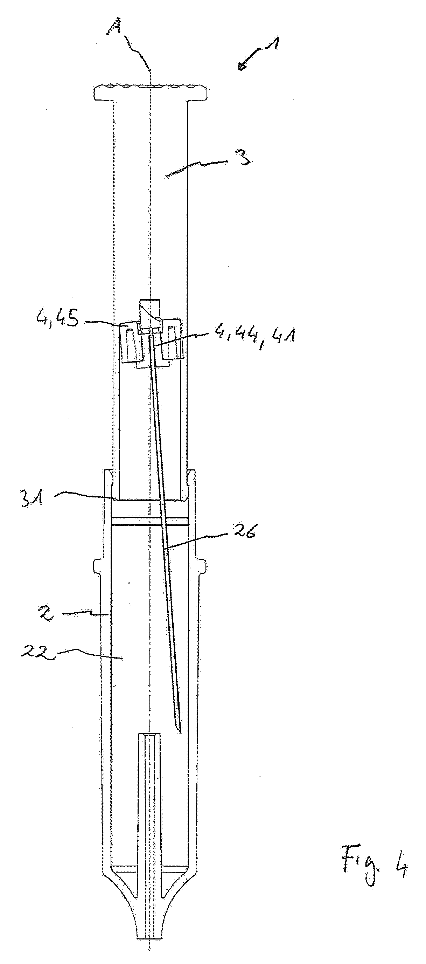

Am hinteren Ende ist die Aufnahmekammer 34 von einer Schrägfläche 55 begrenzt, die mit dem hinteren Ende des Zwischenstücks 4 zusammenwirkt, worauf nachstehend in Verbindung mit Fig. 3 und 4 näher eingegangen wird.

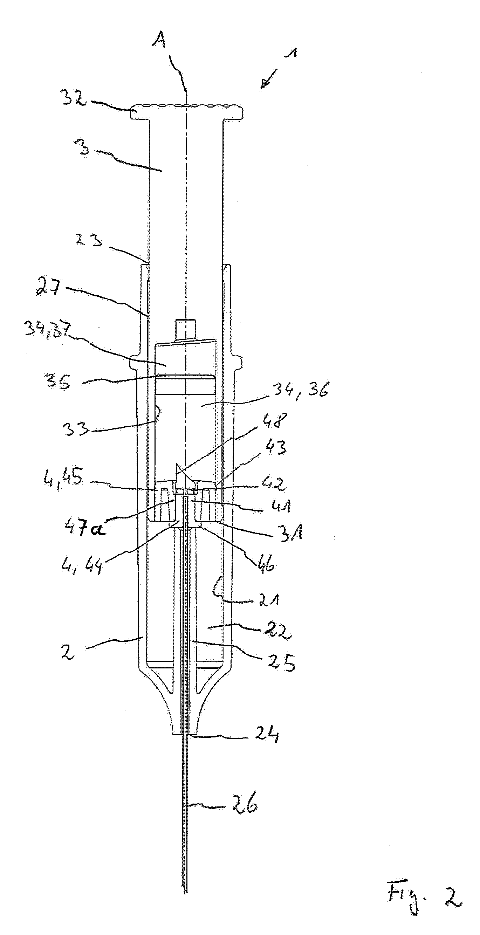

In Fig. 2 ist eine schematische Darstellung des ersten Ausführungsbeispiels in der Freigabestellung dargestellt. Die Freigabestellung entspricht einer aktivierten Stellung, d.h. der Kolben 3 ist mittels einer koaxialen Bewegung mittels eines Kraftübertrags, beispielsweise durch Druck auf das hintere Kolbenende 32, entlang der Achse A im Durchgang 22 in Richtung der vorderen Körperöffnung 24 bewegt worden. Dabei ist eine Halteeinrichtung 27 in Form einer umlaufenden, nach innen vorstehenden Rippe vorgesehen sein, die den Kolben 3 an der Hohlkörperfläche 21 hält, indem eine Reibungskraft erzeugt wird, die es mittels des Kraftübertrages zu überwinden gilt.

In der Freigabestellung ist das innere Element 44 entlang der Achse A in Richtung der vorderen Körperöffnung 24 bewegt worden und am ersten Nadelsitz 25 angeordnet. Dabei liegt das innere Element 44 auf dem ersten Nadelsitz 25 auf und der erste und zweite Nadelsitz 25, 41 bilden gemeinsam einen annähernd durchgehenden Nadelsitz aus. Dabei ist die Nadelkanüle 26, welche in der Speicherstellung vollständig im ersten und zweiten Nadelsitz 25, 41 sowie im Hohlkörper 2 angeordnet ist, entlang der Achse A durch die vordere Körperöffnung 24 aus dem Hohlkörper 2 heraus bewegt worden und befindet sich teilweise ausserhalb des Hohlkörpers 2. Das äussere Element 45 ist ebenfalls entlang der Achse A in Richtung der vorderen Körperöffnung 24 bewegt worden und ist am Anschlag 46 angeordnet, wodurch die Dichtwirkung der Abdichtung 47 des zweiten Dichtbereiches deaktiviert worden ist, so dass in der Freigabestellung die erste Kammer 36 der Aufnahmekammer 34 und der Zuführraum 42 strömungsverbunden sind.

In der Freigabestellung kann das erste Fluid von der ersten Kammer 36 durch den Zuführraum 42 in das innere Element 44 bzw. in eine darin ausgebildete Nadelsitzöffnung und dann in die Nadelkanüle 26 strömen.

Beim anschließenden Übergang in eine Spülstellung (nicht dargestellt) nach Entleeren der ersten Kammer 36 durchsticht der Dorn 48 die Folie, woraufhin die zweite Kammer 37 und der Zuführraum 42 strömungsverbunden sind und durch Austragen des zweiten Fluids, also durch Entleeren der zweiten Kammer 37, durch die Nadelkanüle 26 hindurch ein Spülvorgang durchgeführt werden kann.

Fig. 3 zeigt eine schematische Darstellung des ersten Ausführungsbeispiels nach Austrag des ersten Fluids und des zweiten Fluids. In dieser Endstellung liegt der Kolben 3 mit der am hinteren Ende der Aufnahmekammer 34 ausgebildeten Schräge 55 unter Zwischenlage der aufgebrochenen Folie 35 am Zwischenstück 4 an. Der Kolben 3 ist annähernd vollständig im Durchgang 22 des Hohlkörpers 2 angeordnet, wobei eine weitere Bewegung des Kolbens 3 entlang der Achse A in Richtung der vorderen Körperöffnung 24 verhindert ist. Das Trennelement 35 ist mittels des Dorns 48 aufgebrochen worden und das zweite Fluid ist aus der zweiten Kammer 37 (Fig. 2) über den Zuführraum 42 in die Nadelkanüle 26 geströmt. In axialer Richtung nach hinten über die Schräge 55 hinaus ist eine Vertiefung vorgesehen, in welche der Dorn 48 hineinragt.

Die Schräge 55 strebt danach, das Zwischenstück 4 samt innerem Element 44 und Nadelkanüle 26 schräg zu stellen, was in der Stellung gemäß Fig. 3 aber nicht möglich ist, da sich die Nadelkanüle 26 noch im ersten Nadelsitz 25 befindet und somit nicht ausgelenkt bzw. verschwenkt werden kann.

Fig. 4 zeigt eine schematische Darstellung des ersten Ausführungsbeispiels in einer Einzugsstellung. In der Einzugsstellung ist der Kolben 3 annähernd vollständig aus dem Durchgang 22 des Hohlkörpers 2 herausgezogen, wobei die vordere Kolbenöffnung 31 im Durchgang 22 angeordnet ist. Die Nadelkanüle 26 ist am Zwischenstück 4 über das innere Element 44 am zweiten Nadelsitz 41 derart festgehalten, dass die Nadelkanüle 26 bei der Rückwärtsbewegung des Kolbens 3 mitgezogen wird. Dabei wird die Nadelkanüle 26 vollständig in den Durchgang 22 und aus dem ersten Nadelsitz 25 herausgezogen und somit freigegeben. Das aufgrund der Schräge 55 in eine entsprechende Schrägstellung vorgespannte Zwischenstück 4 kann durch diese Freigabe der Nadelkanüle 26 die Schrägstellung einnehmen und folglich die Nadelkanüle 26 schrägstellen, die somit nicht versehentlich bzw. zufällig wieder in den ersten Nadelsitz 25 einfädeln kann und folglich gewissermaßen im Hohlkörper 2 gefangen ist. Die Nadelkanüle 26 bzw. die Spritze 1 kann daher nicht wiederverwendet werden und es besteht keine Verletzungsgefahr.

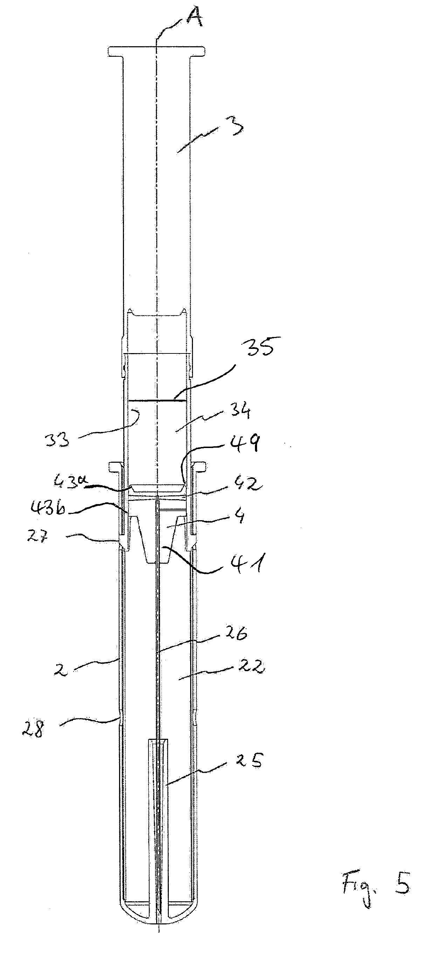

Fig. 5 zeigt eine schematische Darstellung eines zweiten Ausführungsbeispiels einer erfindungsgemäßen Spritze in der Speicherstellung. Der Aufbau der Spritze 1 weist dabei viele Gemeinsamkeiten mit der Spritze 1 aus Fig. 1 bis 4 auf, weshalb im Wesentlichen auf die Unterschiede eingegangen wird.

Das Zwischenstück 4 ist, analog zu Fig. 1 bis 4, in dem Durchgang 22 entlang der Achse A bewegbar angeordnet. Im Zwischenstück 4 ist ein zweiter Nadelsitz 41 ausgebildet, wobei die Nadelkanüle 26 fest im zweiten Nadelsitz 41 gehalten ist. Das Zwischenstück 4 ist im Gegensatz zu dem Ausführungsbeispiel gemäß Fig. 1 bis 4 einteilig. Das einteilige Zwischenstück 4 weist einen Zuführraum 42 auf, der im Zwischenstück 4 ausgebildet ist. In der in Fig. 5 gezeigten Speicherstellung ist der erste Dichtbereich 43a zwischen der Zwischenwand 33 und dem Zwischenstück 4 ausgebildet und bildet eine erste Abdichtung auf der hinteren Seite des Zuführraums 42. Eine zweite Abdichtung 43b ist auf der vorderen Seite des Zuführraums 42 angeordnet. Die vom ersten Dichtbereich 43a gebildete Abdichtung umfasst eine Nut an der Zwischenwand 33 der Aufnahmekammer 34 und eine Ausbuchtung am Zwischenstück 4. Der erste Dichtbereich 43a verhindert, dass in der Speicherstellung Fluid in den Zuführraum 42 strömen kann, während die zweite Abdichtung 43b verhindert, dass Fluid vom Zuführraum 42 in den Durchgang 22 des Hohlkörpers 2 strömen kann. Somit sind die Aufnahmekammer 34 und der Zuführraum 42 in der Speicherstellung nicht strömungsverbunden, d.h. es strömen weder das erste Fluid noch das zweite Fluid von der Aufnahmekammer 34 in den Zuführraum 42.

In der Speicherstellung wird der Kolben 3 von einer Halteeinrichtung 27 gehalten und kann nur mittels eines Kraftübertrags bewegt werden. In einer nicht gezeigten Aktivstellung wird der Kolben 3 mittels einer Aktivierungseinrichtung 28 gehalten und kann nur mittels eines Kraftübertrags in die Freigabestellung bewegt werden. In der Aktivstellung ist die Nadelkanüle 26 ausgefahren. Das hier nur schematisch gezeigte Trennelement 35 wird in diesem zweiten Ausführungsbeispiel mittels einer scharfen umlaufenden Kante 49 des Zwischenstücks 4 aufgebrochen. Es kann eine Schräge entsprechend dem Ausführungsbeispiel der Fig. 1 bis 4 oder ein anderes Mittel zum Verschwenken oder Auslenken der Nadelkanüle 26 vorgesehen sein.

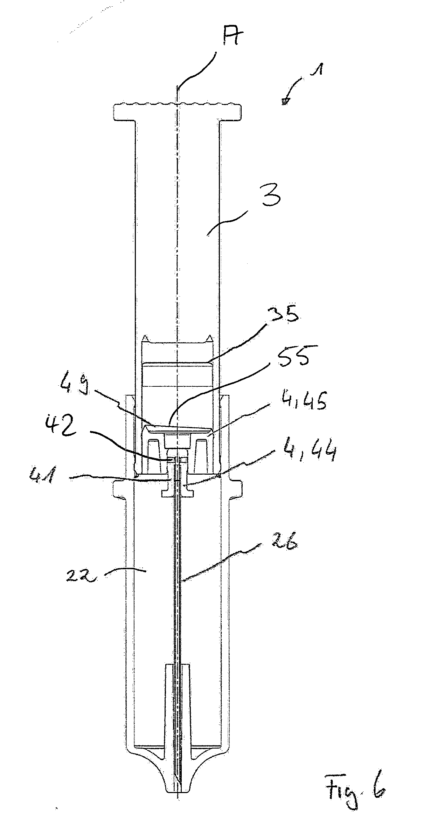

Fig. 6 zeigt eine schematische Darstellung eines dritten Ausführungsbeispiels einer erfindungsgemäßen Spritze in der Speicherstellung. Der Aufbau der Spritze 1 weist dabei viele Gemeinsamkeiten mit der Spritze 1 aus Fig. 1 bis 4 auf, weshalb im Wesentlichen auf die Unterschiede eingegangen wird. Das zweiteilige Zwischenstück 4 ist im Durchgang 22 entlang der Achse A bewegbar angeordnet. Im Zwischenstück 4 ist ein zweiter Nadelsitz 41 ausgebildet, wobei die Nadelkanüle 26 fest im zweiten Nadelsitz 41 gehalten ist. Am äusseren Element 45 ist, in Richtung des hinteren Kolbenendes 32, eine scharfe umlaufende Kante 49 zum Aufbrechen des Trennelements 35 ausgebildet. Die Kante 49 besitzt keine umlaufend konstante Höhe, sondern definiert eine gegenüber der Achse A geneigte Ebene 55, die analog zur Schrägfläche 55 beim Ausführungsbeispiel der Fig. 1 bis 4 für eine Schrägstellung der Nadelkanüle 26 in der Einzugsstellung sorgt.

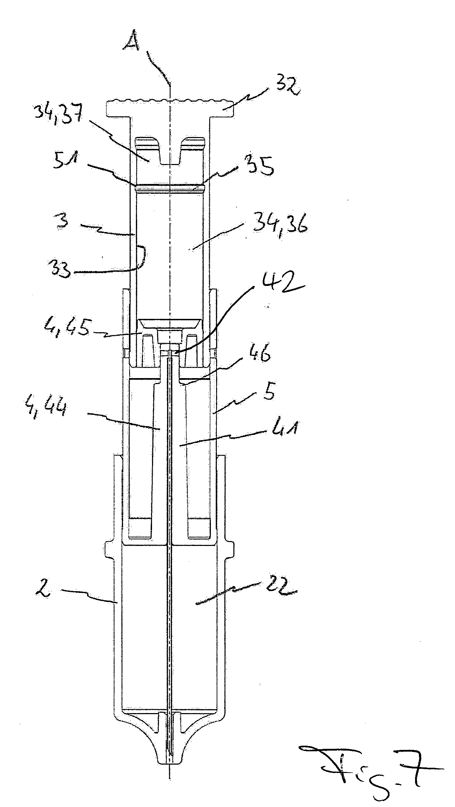

Fig. 7 zeigt eine schematische Darstellung eines vierten Ausführungsbeispiels einer erfindungsgemäßen Spritze in der Speicherstellung. Der Aufbau der Spritze 1 entspricht im Wesentlichen der Spritze 1 aus den Fig. 1 bis 4, weshalb im Wesentlichen auf die Unterschiede eingegangen wird. Das Zwischenstück 4 ist zweiteilig, wobei das innere Element 44 derart ausgestaltet ist, dass der zweite Nadelsitz 41 hohlzylinderförmig ist und zwei unterschiedliche Außendurchmesser aufweist und so den Anschlag 46 für das äussere Element 45 ausbildet. Ausserdem ist das innere Element 44 als ein weiterer Hohlkörper 5 ausgebildet, in welchem der Kolben 3 angeordnet ist. Das insgesamt einstückig ausgebildete innere Element 44 umfasst folglich den radial innen liegenden Nadelsitz 41 und einen äußeren, den Hohlkörper 5 definierenden Teil, der konzentrisch zum Nadelsitz 41 angeordnet ist und den Kolben 3 aufnimmt. Das innere Element 44 weist keinen Dorn 48 auf.

Der weitere Hohlkörper 5 ist im Durchgang 22 des Hohlkörpers 2 angeordnet. Die koaxiale Relativbewegung des Kolbens 3 erfolgt somit entlang der Achse A im weiteren Hohlkörper 5 des inneren Elements 44, welches sich wiederum entlang der Achse A im Hohlkörper 2 bewegt.

Das Trennelement 35 ist als ein Stopfen ausgebildet und entlang der Achse A beweglich in der Aufnahmekammer 34 angeordnet. Zum Entleeren der zweiten Kammer 37 kann das Trennelement 35 mittels des äußeren Elements 45 des Zwischenstücks 4 verschoben werden. Alternativ kann vorgesehen sein, dass das z.B. als Folie ausgebildete Trennelement 35 mittels des äußeren Elementes 45 aufgebrochen wird, insbesondere mittels einer am äußeren Element 45 ausgebildeten scharfen Kante.

Ausserdem ist zwischen der Zwischenwand 33 und dem Trennelement 35 ein in der Speicherstellung bzw. Freigabestellung wirksamer Dichtbereich 51 derart ausgebildet und angeordnet, dass die zweite Kammer 37 und der Zuführraum 42 keine Strömungsverbindung aufweisen, ebenso wie die zweite Kammer 37 und die erste Kammer 36 keine Strömungsverbindung aufweisen. Der Dichtbereich 51 umfasst eine Nut in der Zwischenwand 33 und eine dazu komplementäre Ausbuchtung im Trennelement 35. In der Spülstellung ist der Dichtbereich 51 deaktiviert und die zweite Kammer 37 und der Zuführraum 42 sind strömungsverbunden.

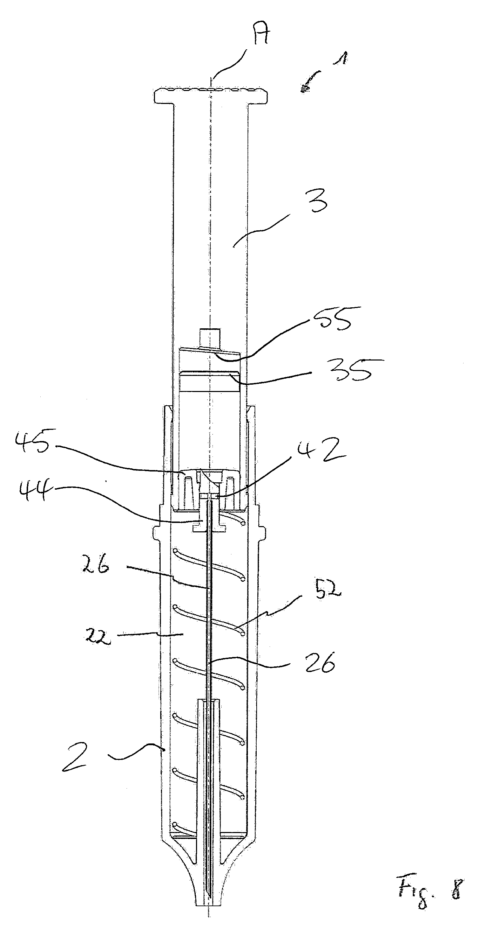

Fig. 8 zeigt eine schematische Darstellung eines fünften Ausführungsbeispiels einer erfindungsgemäßen Spritze 1 in der Speicherstellung. Der Aufbau der Spritze 1 entspricht im Wesentlichen der Spritze 1 aus den Fig. 1 bis 4, weshalb im Wesentlichen auf die Unterschiede eingegangen wird. Um beim Übergang in die Einzugsstellung dafür zu sorgen, dass die Nadelkanüle 26 vollständig in den Durchgang 22 gezogen wird, ist im Durchgang 22 eine Feder 52 angeordnet, gegen deren Rückstellkraft der Kolben 3 ausgehend von der Speicherstellung nach vorne gedrückt werden muss. Die Feder 52 bewirkt oder unterstützt folglich nach bestimmungsgemäßem Gebrauch der Spritze 1 den Übergang in die Einzugsstellung.

### Bezugszeichenliste

- 1: Spritze
- 2: Hohlkörper
- 3: Kolben
- 4: Zwischenstück
- 5: Hohlkörper
- 21: Hohlkörperfläche
- 22: Durchgang
- 23: hintere Körperöffnung
- 24: vordere Körperöffnung
- 25: erster Nadelsitz
- 26: Nadelkanüle
- 31: vordere Kolbenöffnung
- 32: hinteres Kolbenende
- 33: Zwischenwand
- 34: Aufnahmekammer
- 35: Trennelement, Folie
- 36: erste Kammer
- 37: zweite Kammer
- 41: zweiter Nadelsitz
- 42: Zuführraum
- 43: erster Dichtbereich
- 43a: erster Dichtbereich
- 44: inneres Element
- 45: äußeres Element
- 46: Anschlag
- 47: Abdichtung
- 47a: Abdichtung
- 48: Dorn
- 49: Kante
- 51: Dichtbereich
- 52: Feder
- 55: Schwenk- bzw. Auslenkmittel, Schrägfläche, geneigte Ebene

- A: Achse

## Patentansprüche

1. Spritze (1), umfassend
- einen Hohlkörper (2), der eine umschließende Hohlkörperfläche (21) aufweist und darin einen Durchgang (22) ausbildet, wobei in eine axiale Längsrichtung des Hohlkörpers (2) eine Achse (A) ausgebildet ist und der Durchgang (22) eine hintere Körperöffnung (23) und eine vordere Körperöffnung (24) aufweist, die entgegengesetzt zueinander in der Längsrichtung angeordnet sind, wobei an der vorderen Körperöffnung (24) ein erster Nadelsitz (25) ausgebildet ist, in welchem eine Nadelkanüle (26) angeordnet und relativ zu welchem die Nadelkanüle (26) entlang der Achse (A) bewegbar ist,
- einen Kolben (3), der im Hohlkörper (2) angeordnet und in dem Durchgang (22) entlang der Achse (A) bewegbar ist, wobei der Kolben (3) eine vordere Kolbenöffnung (31), ein hinteres Kolbenende (32) und eine umschließende Zwischenwand (33) umfasst, die zwischen der vorderen Kolbenöffnung (31) und dem hinteren Kolbenende (32) angeordnet ist und die eine Aufnahmekammer (34) zur Aufnahme zumindest eines Fluids ausbildet, und
- ein Zwischenstück (4), das in der Aufnahmekammer (34) angeordnet und entlang der Achse (A) bewegbar ist, wobei zwischen der Zwischenwand (33) und dem Zwischenstück (4) ein erster Dichtbereich (43; 43a) wirksam ist, der die Aufnahmekammer (34) in einer Speicherstellung abdichtet,
wobei in dem Zwischenstück (4) ein zweiter Nadelsitz (41) und ein Zuführraum (42) ausgebildet sind, wobei der zweite Nadelsitz (41) und der Zuführraum (42) strömungsverbunden sind,
wobei der Kolben (3), der Hohlkörper (2) und das Zwischenstück (4) mittels einer koaxialen Relativbewegung ausgehend von der Speicherstellung in eine Freigabestellung bewegbar sind, und
wobei in der Speicherstellung zwischen dem Zuführraum (42) und der Aufnahmekammer (34) eine Abdichtung (47; 43a) vorgesehen ist, die durch die koaxiale Relativbewegung aufhebbar ist, so dass in der Freigabestellung die Aufnahmekammer (34) und der Zuführraum (42) strömungsverbunden sind.

2. Spritze nach Anspruch 1, wobei das Zwischenstück (4) ein äusseres Element (45) und ein inneres Element (44) umfasst und das innere Element (44) zum Aufheben der Abdichtung (47) axial relativ zu dem äusseren Element (45) bewegbar ist, wobei insbesondere das innere Element (44) den zweiten Nadelsitz (41) umfasst.

3. Spritze nach Anspruch 2, wobei das innere Element (44) einen Anschlag (46) für das äussere Element (45) umfasst, wobei insbesondere das innere Element (44) mit dem Anschlag (46) derart angeordnet ist, dass eine axiale Bewegung des inneren Elementes (44) relativ zu dem äusseren Element (45) in Richtung des hinteren Körperendes (23) durch den Anschlag (46) begrenzt ist.

4. Spritze nach Anspruch 2 oder 3, wobei die Abdichtung (47) zwischen dem inneren Element (44) und dem äusseren Element (45) ausgebildet ist.

5. Spritze nach einem der vorangehenden Ansprüche, wobei die Aufnahmekammer (34) mittels eines Trennelements (35) in eine erste Kammer (36) und eine zweite Kammer (37) unterteilt ist, wobei die erste Kammer (36) zwischen der zweiten Kammer (37) und dem Zwischenstück (4) gelegen ist, wobei insbesondere in einer Spülstellung die zweite Kammer (37) und der Zuführraum (42) strömungsverbunden sind.

6. Spritze nach Anspruch 5, wobei in der Speicherstellung zwischen der Zwischenwand (33) und dem Trennelement (35) ein Dichtbereich (51) derart ausgebildet und angeordnet ist, dass die zweite Kammer (37) und der Zuführraum (42) keine Strömungsverbindung aufweisen.

7. Spritze nach Anspruch 5 oder 6, wobei das Trennelement (35) als eine Folie ausgebildet ist.

8. Spritze nach einem der Ansprüche 5 bis 7, wobei das Zwischenstück (4) in Richtung des hinteren Körperendes (23) als ein Dorn (48) zum Aufbrechen des Trennelements (35) ausgebildet ist.

9. Spritze nach einem der Ansprüche 5 bis 9, wobei das Trennelement (35) als ein Stopfen ausgebildet und entlang der Achse (A) beweglich in der Aufnahmekammer (34) angeordnet ist.

10. Spritze nach einem der vorangehenden Ansprüche, wobei das Zwischenstück (4) einteilig ausgebildet und zum Aufheben der Abdichtung (43a) axial relativ zum Kolben (3) bewegbar ist.

11. Spritze nach einem der vorangehenden Ansprüche, wobei das innere Element (44) als ein weiterer Hohlkörper (5) ausgebildet ist und der Kolben (3) beweglich im weiteren Hohlkörper (5) angeordnet ist.

12. Spritze nach einem der vorangehenden Ansprüche, wobei im Durchgang (22) eine Feder (52) angeordnet ist, gegen deren Rückstellkraft der Kolben (3) in Richtung der vorderen Körperöffnung (24) des Hohlkörpers (2) bewegbar ist.

13. Spritze nach einem der vorangehenden Ansprüche, wobei die Aufnahmekammer (34) wenigstens ein Fluid enthält, insbesondere eine Flüssigkeit enthaltend wenigstens ein zu verabreichendes Medikament in einer ersten Kammer (36) der Aufnahmekammer (34) und eine Spüllösung in einer zweiten Kammer (37) der Aufnahmekammer (34).

14. Spritze nach einem der vorangehenden Ansprüche, wobei Mittel (55) zum automatischen seitlichen Verschwenken oder Auslenken des Zwischenstücks (4) in einer Endstellung vorgesehen sind, wobei insbesondere die Mittel (55) zwischen einem hinteren Ende der Aufnahmekammer (34) und dem Zwischenstück (4) wirksam sind und insbesondere wenigstens eine Schrägfläche (55) umfassen oder wenigstens eine geneigte Ebene (55) definieren.

15. Verwendung einer Spritze nach einem der vorangehenden Ansprüche zum Ausgeben eines bereits in der Aufnahmekammer (34) enthaltenen Fluids ohne Aufziehen des Fluids, insbesondere zum Injizieren einer Flüssigkeit enthaltend wenigstens ein zu verabreichendes Medikament, wobei insbesondere in einer ersten Phase eine Flüssigkeit enthaltend wenigstens ein zu verabreichendes Medikament aus einer ersten Kammer (36) der Aufnahmekammer (34) ausgegeben wird und anschließend in einer zweiten Phase eine Spüllösung aus einer zweiten Kammer (37) der Aufnahmekammer (34) ausgegeben wird.
